# EUROPEAN PATENT APPLICATION

(11) **EP 1 388 320 A1**
(43) Date of publication of application: **11.02.2004**
(21) Application number: 02017600.4
(22) Date of filing: 07.08.2002
(51) Int. Cl.: A61B 5/024, A61B 5/00

(54) **Pulsimeter combined with portable communication device**

(71) Applicant: Sony Ericsson Mobile Communications AB, 221 88 Lund (SE)
(72) Inventor: Frohlund, Stig, 281 38 Hässleholm (SE)
(74) Representative: Dahnér, Christer

(57) **Abstract**

The present invention relates to a portable electronic device and a pulse sensing and information presenting device connectable to a portable communication device and comprising: a pulse sensing unit (30) for providing pulse measurements to be submitted to a pulse determining unit for presenting pulse information to the user, and a first information presentation unit (28) provided adjacent the pulse sensing unit for presenting information from an information processing unit to the user, wherein the combination of first information presentation unit and pulse sensing unit is attachable to the human body.

## Description

### TECHNICAL FIELD OF THE INVENTION

The present invention relates to the field of portable electronic devices and more particularly to a portable electronic device comprising a pulsimeter and another information presentation device as well as to a pulse sensing and information presentation device.

### DESCRIPTION OF RELATED ART

It is known to provide pulsimeters for measuring the pulse of a user and presenting the pulse to the user. For instance one such pulsimeter includes a handheld device where a user puts his/her fingertip on a sensor that feels the pulse beat and presents it as buzzer sounds. This device also presents a number of beats per minute in an LCD display.

Users are however often interested in measuring the pulse, while for instance exercising, in which case it is hard to do this with the device described above. Users furthermore often like to listen to music or to be able to talk on a phone while exercising.

There thus exists a need for measuring the pulse during physical activity like while for instance exercising. This need is often combined with the need to for example listening to music or being able to talk on a phone during the physical activity.

### SUMMARY OF THE INVENTION

The present invention is directed towards solving the problem of safely measuring the pulse of a user during physical activity while at the same time allowing the user to be able to engage in other activities like listening to music or receive phone calls in a portable electronic device and without adding new devices, which the user has to keep track of.

According to a first aspect of the present invention this problem is solved by a portable electronic device including: a pulse sensing unit providing pulse measurements, a pulse determining unit, an information processing unit and a first information presentation unit provided adjacent the pulse sensing unit, which first information presentation unit can present information from the information processing unit to the user, wherein the combination of first information presentation unit and pulse sensing unit is attachable to the human body.

A second aspect of the present invention is directed towards a portable electronic device including the features of the first aspect, and further including a fastener for fastening the combination to the body.

A third aspect of the present invention is directed towards a portable electronic device including the features of the first aspect, wherein the first information presentation unit is a sound presentation unit.

The present invention is furthermore directed to measuring the pulse at a part of the human body, which is much better than the fingertip while at the same time allowing the user to be able to engage in the other activities.

According to a fourth aspect of the present invention, this is achieved by a portable electronic device including the features of the third aspect, wherein the sound presentation unit is arranged to be connected to the ear of a user.

A fifth aspect of the present invention is directed towards a portable electronic device including the features of the third aspect, wherein the pulse determining unit presents the pulse in the form of audible pulse beats on the first information presentation unit.

A sixth aspect of the present invention is directed towards a portable electronic device including the features of the first aspect, wherein the pulse determining unit and the information processing unit can present information to the user simultaneously via the first information presentation unit.

A seventh aspect of the present invention is directed towards a portable electronic device including the features of the sixth aspect, further including a second information presentation unit, wherein both the pulse determining unit and the information processing unit can provide presentation of information to the user either on the first information presentation unit or the second information presentation unit according to the choice of the user.

An eighth aspect of the present invention is directed towards a portable electronic device including the features of the first aspect, and further comprising a third information presentation unit in the form of a display for presenting further graphical information to the user about the pulse.

A ninth aspect of the present invention is directed towards a portable electronic device including the features of the eighth aspect, wherein the pulse determining unit presents statistical data of the pulse over a period of time on the third information presentation unit.

A tenth aspect of the present invention is directed towards a portable electronic device including the features of the first aspect, which is at least partly provided in a cellular phone.

An eleventh aspect of the present invention is directed towards a portable electronic device including the features of the tenth aspect, wherein the pulse determining unit is arranged to generate an alarm if the pulse is higher than a certain level.

A twelfth aspect of the present invention is directed towards a portable electronic device including the features of the eleventh aspect, wherein the pulse determining unit is further arranged to prompt the user to verify the alarm and dial an emergency number in case the user does not verify the alarm.

A thirteenth aspect of the present invention is directed towards a portable electronic device including the features of the first aspect, wherein at least the pulse sensing unit and first information presentation unit are provided in the form of an extra device pluggable into another device, from which other device a user can control pulse sensing and information presentation.

The present invention is furthermore directed towards the problem of providing a pulse sensing and information presenting device, in which safe measurement of the pulse of a user during physical activity can be performed while at the same time allowing the user to be able to engage in other activities like listening to music or receive phone calls.

According to a fourteenth aspect of the present invention, this is solved by a pulse sensing and information presenting device which is connectable to a portable electronic device, said pulse sensing and information presenting device comprising: a pulse sensing unit for providing pulse measurements to be submitted to a pulse determining unit for presenting pulse information to the user, and a first information presentation unit provided adjacent the pulse sensing unit for presenting information from an information processing unit to the user, wherein the combination of first information presentation unit and pulse sensing unit is attachable to the human body.

A fifteenth aspect of the present invention is directed towards a pulse sensing and information presenting device including the features of the fourteenth aspect, and further comprising a pulse determining unit in order to determine and make possible presentation of pulse information to said user.

A sixteenth aspect of the present invention is directed towards a pulse sensing and information presenting device including the features of the fourteenth aspect, and further comprising the information processing unit.

The invention has the following advantages. No extra gripping device or fastener is needed for the pulse sensing unit, since it takes advantage of the ways of gripping, which is already in place for the first information presentation unit. Yet another advantage is that a user only has to keep track of one device connected to his body instead of two different ones, which can be a problem during exercise. The fourth aspect of the present invention furthermore has the following advantages: By selecting the ear for measuring the pulse, a much better place for measuring the pulse is obtained compared with for instance the fingertip. The ear is a part of the human body, which gives very good results for pulse measurements. By selecting the ear it is furthermore possible to combine it with listening to other media as well as possibly the pulse beat. If an earphone is used there is also a good and stable sensor effect obtained by the present sensor, since it uses the griping force of the earphone.

It should be emphasized that the term "comprises/comprising" when used in this specification is taken to specify the presence of stated features, integers, steps or components, but does not preclude the presence or addition of one or more other features, integers, steps, components or groups thereof.

### BRIEF DESCRIPTION OF THE DRAWINGS

The present invention will now be described in more detail in relation to the enclosed drawings, in which:
fig. 1 shows a portable electronic device in the form of a cellular phone to which is connected a plug-in device,
fig. 2 shows a block schematic of relevant units in the phone in fig. 1 as well as the connected plug-in device,
fig. 3 shows a schematic drawing of an earphone part of the plug-in device connected to the ear of a user,
fig. 4 shows a front view of the earphone in fig. 3, and
fig. 5 shows a sectional view of the earphone in fig. 4 where the section has been taken along line A-B of fig. 4.

### DETAILED DESCRIPTION OF EMBODIMENTS

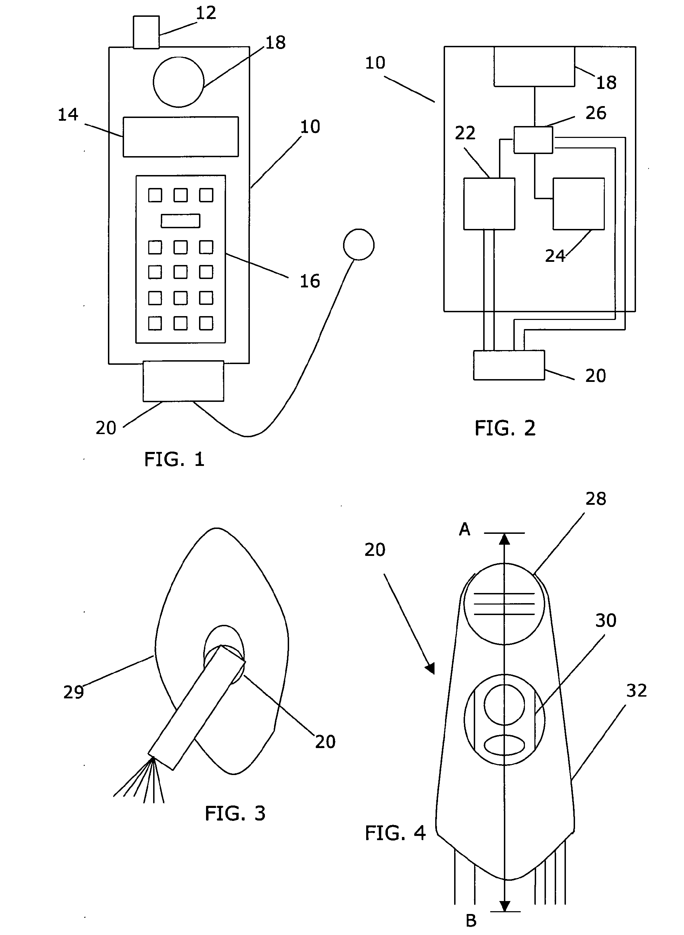

The present invention is directed towards a portable electronic device and a pulse sensing and information presenting device. A portable electronic device 10 is shown in fig. 1. In the preferred embodiment the device is a cellular phone 10 having an antenna 12, a display 14, a keypad 16 including a number of keys, and a speaker 18. The phone is provided with a system connector (not shown) into which a plug-in device 20 according to the invention is plugged. The portable electronic device according to the invention then comprises the phone 10 and the plug-in device 20, while the pulse sensing and information presenting device according to the invention then comprises the plug-in device 20 but not the phone. The plug-in device 20 comprises an earphone, which will be described in more detail later on, as well as a pulse sensing unit. The earphone is here a first information presentation unit, the speaker 18 a second information presentation unit and the display 14 a third information presentation unit. The first and second information presentation units are in this embodiment thus sound presentation units. The plug-in device 20 can be connected to the ear of a user. The keypad 16 is used for entering information such as selection of functions and responding to prompts and the display 14 is used for displaying functions and prompts to a user of the phone. A cellular phone is just one example of a device in which the invention can be implemented. The invention can for instance also be used in a PDA (personal digital assistant), a palm top computer, a portable music playing device such as a CD player or an MP3 player or a cassette player. It can also be used in a portable radio. What is important is that it is possible to combine the pulsimeter according to the invention with some other type of information processing unit.

Fig. 2 shows a block schematic of some relevant parts of the interior of the phone according to the present invention. The cellular phone 10 includes a pulse determining unit 22 connected to the pulse sensing unit (not shown) of the plug-in device 20. The phone also includes an information processing unit 24. Both the information processing unit 24 and the pulse determining unit 22 are connected to a switch 26, which switch can connect both the information processing unit 24 and the pulse determining unit 22 to the speaker 18 or to the earphone (not shown) via the plug-in device 20. The information processing unit 24 is a unit which can present information for example in the form of music and is here an MP3 player, although it should be realised that it can be some other type of unit as well, such as a unit for responding to phone calls.

Fig. 3 shows an upper part of the plug-in device 20 connected to an ear 29 of a user in order to show the use of the invention. The upper part is connected to the plug-in part via a cable including six wires. Fig. 4 shows a front view of the upper part of the plug-in device 20, where an earphone 28 and a pulse sensing unit 30 are partly covered by a cover 32.

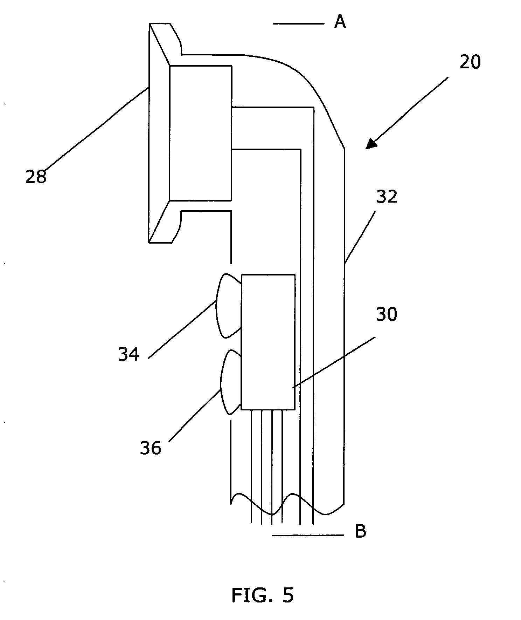

Fig. 5 shows a sectional view of the upper part of the plug-in device 20, where the section has been taken along line A-B of fig. 4. Fig. 5 shows the earphone 28 provided above the pulse sensing unit 30. From fig. 4 and 5 it can be seen that the speaker 28 of the earphone has two wires for connecting to the phone. The pulse sensing unit 30 has four wires, which connect to the pulse determining unit of the phone 10. The pulse sensing unit 30 includes an IR (Infrared) transmitter 34 and IR receiver 36, the functioning of which will be described shortly. Two of the wires are intended for the IR transmitter 34 and two wires for the IR receiver 36. The number of wires can be varied though. There might be only three wires to the pulse sensing unit 30. In this latter case the IR transmitter 34 and IR receiver 36 share a common ground wire.

Now the functioning of the present invention will be explained. A user of the phone 10 together with the plug-in device 20, will put the upper part of the plug-in device 20, i.e. the earphone 28 into the ear. Thereby the pulse sensing unit 30 is held in a measuring position close to the ear of the user or rather the earlobe of the user. This is accomplished by the earphone grabbing around the wing of the ear opening, which gives a better and more stable sensor effect than with a finger tip. The shape of the earphone here provides a fastener for holding the pulse sensing unit 30 and earphone 28 to the body of the user. The MP3 player can then also be activated such that the user can use the phone as a music playing device to be used for instance when out jogging. The user can then also start the pulsimeter, for instance via selection of pulse metering in a suitable menu of the phone, i.e. by actuating ON/OFF buttons of the phone or other similar buttons. The MP3 player can be actuated in a similar fashion. When the user has done this, there is first a calibration of the pulse sensing unit 30. The pulse sensing unit 30 therefore senses how strong the pulse signals need to be to be able to pick up a pulse and calibrates the pulse sensing unit accordingly. Thereafter the pulsimeter starts measuring the pulse via the pulse sensing unit 30. The pulse sensing unit senses the blood pressure via the IR transmitter 34 and receiver 36 and returns the measurement results to the pulse determining unit 22. The pulse determining unit 22 then determines the pulse of the user. The pulse can thereafter be presented to the user as a number of pulse beats, which are presented to the user together with the music being played. The pulse beats are presented as a number of tones having lower pitch than what the music played has. These pulse beats are preferably sent via the same earphone the user is already wearing, but they can also be sent via the speaker of the phone if the user so wishes. The pulse determining unit 22 can furthermore also present the pulse beats as for example number of beats per minute presented on the display or number of beats according to any other suitable time period. In this case the pulse determining unit 22 has to calculate the value based on a number of measurements. The pulse determining unit 22 can also measure the pulse during a certain longer time, for instance during a jog, and then present the variations later. The user can then store different pulse histories and see how much the pulse has been varied for different occasions. A user can in this case see how his/her health is progressing by comparing changes in pulse for different time periods. This is then presented on the display or can be exported to a computer or other suitable device, where the user can watch his pulse history.

According to another aspect of the invention, the pulsimeter measures the pulse and if the pulse is above a certain limit, which can be a too high pulse level of the user, the phone prompts the user to respond. If the user is not responding the phone then makes an emergency call automatically.

By selecting the ear for measuring the pulse, a much better place for measuring the pulse is obtained compared with for instance the fingertip. The ear is a part of the human body, which gives very good results for pulse measurements. By selecting the ear it is furthermore possible to combine it with listening to other media as well as possibly the pulse beat. Because of the use of an earphone there is also a good and stable sensor effect obtained by the present sensor, since it uses the griping force of the earphone. Another advantage is that no extra fastener or gripping device is needed for the pulse sensing unit, since it takes advantage of the earphone, which is already in place. Yet another advantage is that a user only has to keep track of one device connected to his body instead of two different ones, which can be a problem during exercise.

The invention can be varied in many ways. For instance the plug-in device can include the pulse determining unit. The plug-in device would then use the portable electronic device only for help in presenting the information. It is also possible to do without a plug-in device adapted to the system connector, provided there are separate connections available for the pulse sensing unit and the earphone. The pulsimeter and earphone are then sold either as a separate unit connectable to a portable electronic device or as an accessory provided together with the portable electronic device.

It is also possible to only present the pulse as audible pulse beats or as graphical information.

The earphone is one sound presentation unit that can be used. Another type is for example a headset or headphone covering both ears, but then only the part covering one ear would normally include a pulse sensing unit. Another possible variation is to provide the pulse sensing unit on another part of the body, like the wrist and combine it with a display or a sound presenting unit in the form of a speaker. This pulse sensing and information presenting device can then be held to the body by a fastener in the form of a wrist band.

It should be realised that the present invention is only intended to be limited by the enclosed claims.

## Claims

1. Portable electronic device (10, 20) including:
a pulse sensing unit (30) providing pulse measurements,
a pulse determining unit (22),
an information processing unit (24), and
a first information presentation unit (28) provided adjacent the pulse sensing unit, which first information presentation unit can present information from the information processing unit to the user,
wherein the combination of first information presentation unit and pulse sensing unit is attachable to the human body (29).

2. Portable electronic device according to claim 1, further comprising a fastener for fastening the combination to the body.

3. Portable electronic device according to claim 1 or 2, wherein the first information presentation unit (28) is a sound presentation unit.

4. Portable electronic device according to claim 3, wherein the sound presentation unit is arranged to be connected to the ear (29) of a user.

5. Portable electronic device according to claim 3 or 4, wherein the pulse determining unit presents the pulse in the form of audible pulse beats on the first information presentation unit.

6. Portable electronic device according to any previous claim, wherein the pulse determining unit and the information processing unit can present information to the user simultaneously via the first information presentation unit.

7. Portable electronic device according to claim 6, further including a second information presentation unit (18), wherein both the pulse determining unit and the information processing unit can provide presentation of information to the user either on the first information presentation unit or the second information presentation unit according to the choice of the user.

8. Portable electronic device according to any previous claim, further comprising a third information presentation unit (14) in the form of a display for presenting further graphical information to the user about the pulse.

9. Portable electronic device according to claim 8, wherein the pulse determining unit presents statistical data of the pulse over a period of time on the third information presentation unit.

10. Portable electronic device according to any previous claim, at least partly provided in a cellular phone (10).

11. Portable electronic device according to claim 10, wherein the pulse determining unit is arranged to generate an alarm if the pulse is higher than a certain level.

12. Portable electronic device according to claim 11, wherein the pulse determining unit is further arranged to prompt the user to verify the alarm and dial an emergency number in case the user does not verify the alarm.

13. Portable electronic device according to any previous claim, wherein at least the pulse sensing unit and first information presentation unit are provided in the form of an extra device (20) pluggable into another device (10), from which other device a user can control pulse sensing and information presentation.

14. Pulse sensing and information presenting device (20) which is connectable to a portable electronic device (10) said pulse sensing and information presenting device comprising:
a pulse sensing unit (30) for providing pulse measurements to be submitted to a pulse determining unit (20) for presenting pulse information to the user, and
a first information presentation unit (28) provided adjacent the pulse sensing unit for presenting information from an information processing unit (24) to the user,
wherein the combination of first information presentation unit and pulse sensing unit is attachable to the human body (29).

15. Pulse sensing and information presenting device according to claim 14, further comprising a pulse determining unit in order to determine and make possible presentation of pulse information to said user.

16. Pulse sensing and information presenting device according to claim 14 or 15, further comprising the information processing unit.
